# EUROPEAN PATENT SPECIFICATION

(11) **EP 4 137 080 B1**
(45) Date of publication and mention of the grant of the patent: **07.08.2024**
(21) Application number: 22190351.1
(22) Date of filing: 15.08.2022
(51) Int. Cl.: A61B 18/14, A61B 5/00, A61B 18/00

(54) **CATHETER HAVING ELECTRODES WITH ADJUSTABLE SIZE**
KATHETER MIT ELEKTRODEN MIT EINSTELLBARER GRÖSSE
CATHÉTER DOTÉ D'ÉLECTRODES À TAILLE RÉGLABLE

(30) Priority: 16.08.2021 US 202117403009
(43) Date of publication of application: 22.02.2023
(73) Proprietor: Biosense Webster (Israel) Ltd., Yokneam 2066717 (IL)
(72) Inventor: ALTMANN, Andres Claudio, 2066717 Yokneam (IL)
(74) Representative: Carpmaels & Ransford LLP

(56) References cited:
- EP-A1- 3 141 183
- EP-A1- 3 335 626
- EP-A2- 3 015 064

## Description

### FIELD OF THE INVENTION

The present invention relates generally to medical catheters, and particularly to a catheter having electrodes with an adjustable size.

### BACKGROUND OF THE INVENTION

Various techniques for increasing the functionality of expandable catheters have been published.

For example, European Patent Application EP3141181A1 describes an ablation catheter having an elongated body member, a basket including a plurality of radially expanding splines and a first electrode located on one of the radially expanding splines. The first electrode configured to apply energy to tissue adjacent to the radially expanding splines during an ablation procedure. At least one of the radially expanding splines includes at least one aperture, a lumen, an optical emitting element inside the lumen and an optical receiving element inside the lumen. The optical emitting element is configured to emit optical radiation through the aperture and into adjacent tissue during the ablation procedure while the optical receiving element is configured to collect optical radiation through the aperture during the ablation procedure. The optical radiation indicates characteristics of adjacent tissue during the ablation procedure.

U.S. Patent Application Publication No. 2020/0206461 describes a system that includes an expandable distal-end assembly, a proximal position sensor, a distal position sensor, and a processor. The expandable distal-end assembly is coupled to a distal end of a shaft for insertion into a cavity of an organ of a patient. The proximal and distal position sensors are located at a proximal end and a distal end of the distal-end assembly, respectively. The processor is configured to estimate a position and a longitudinal direction of the proximal sensor, and a position of the distal sensor, all in a coordinate system used by the processor. The processor is further configured to project the estimated position of the distal sensor on an axis defined by the estimated longitudinal direction, and to calculate an elongation of the distal-end assembly by calculating a distance between the estimated position of the proximal sensor and the projected position of the distal sensor. EP3141183A1 describes a basket catheter with individual spine control.

### SUMMARY OF THE INVENTION

The invention is defined by the claims.

In accordance with an embodiment of the present invention, a catheter is provided that includes: (i) a shaft for insertion into an organ of a patient, (ii) an expandable distal-end assembly, which is coupled to the shaft and includes multiple splines, at least a given spline among the multiple splines includes an electrode that is configured to be placed in contact with tissue of the organ, and (iii) a tube, which is configured to be threaded over at least a portion of the expandable distal-end assembly, and is movable along the distal-end assembly for controlling at least a size of a section of the electrode in contact with the tissue, the tube has a stopper for limiting a movement of the tube relative to the distal-end assembly.

In some embodiments, the tube has at least a channel for controlling a position of at least the given spline along a perimeter of the tube. In other embodiments, the splines are coupled between a distal apex and a proximal apex of the distal-end assembly, and the tube is configured to be moved: (i) toward the proximal apex for increasing the size of the section, and (ii) toward the distal apex for reducing the size of the section.

There is further provided, in accordance with an embodiment of the present invention, a method for producing a catheter, the method includes coupling, to a shaft, an expandable distal-end assembly, which includes multiple splines, and at least a given spline among the multiple splines includes an electrode that is configured to be placed in contact with tissue of an organ of a patient. A tube that is movable for controlling at least a size of a section of the electrode in contact with the tissue, is threaded over at least a portion of the expandable distal-end assembly, the tube has a stopper for limiting a movement of the tube relative to the distal-end assembly.

In some embodiments, the tube has at least a channel for controlling a position of at least one of the splines along a perimeter of the tube. In other embodiments, the splines are coupled between a distal apex and a proximal apex of the distal-end assembly, and the stopper is configured to limit the movement of the tube between the distal apex and the proximal apex for controlling at least the size of the section. In yet other embodiments, the method includes coupling to the tube an additional electrode, which is not intended to be placed in contact with the tissue, so as to one or both: (i) apply a unipolar ablation signal between the electrode and the additional electrode, and (ii) sense a unipolar EA signal between the electrode and the additional electrode.

### BRIEF DESCRIPTION OF THE DRAWINGS

The present invention will be more fully understood from the following detailed description of the embodiments thereof, taken together with the drawings in which:
Fig. 1 is a schematic, pictorial illustration of a catheter-based position-tracking and ablation system, in accordance with an exemplary embodiment of the present invention;
Figs. 2A and 2B are schematic, side views of an expandable distal-end assembly of a catheter and a movable tube for controlling the size of one or more electrodes of the distal-end assembly placed in contact with tissue, in accordance with exemplary embodiments of the present invention;
Fig. 3 is a flow chart that schematically illustrates a method, which is not claimed (but is considered as useful for understanding the invention) for controlling the size of one or more electrodes of an expandable distal-end assembly that are placed in contact with tissue; and
Fig. 4 is a flow chart that schematically illustrates a method for producing a catheter comprising an expandable distal-end assembly having electrodes and a tube for controlling the size of the electrodes placed in contact with tissue, in accordance with an exemplary embodiment of the present invention.

### DETAILED DESCRIPTION OF EMBODIMENTS

### OVERVIEW

Electrophysiological procedures typically require sensing electro-anatomical (EA) signals in tissue of a patient organ, e.g., a heart, for mapping the EA and identifying locations in tissue that may generate arrhythmias in the heart. Subsequently, if needed based on the EA mapping, the electrophysiological procedure may require application of ablation signals to the aforementioned locations of the tissue in question. The EA mapping typically requires high-resolution, which is obtained by using electrodes having a small size (e.g., about 2.5 mm). The ablation, however, may require electrodes having a larger size (e.g., about 10 mm).

In principle, it is possible to use a catheter having one or more sensing electrodes for the EA sensing, and one or more ablation electrodes for the tissue ablation. Alternatively, it is possible to use different catheters, one for sensing and another for ablation. The first option requires a complex catheter, which may cause operational challenges (e.g., by coupling different type of electrode(s) to the tissue during the EA mapping and tissue ablation). The second option requires insertion of two different catheters to the patient heart. In summary, both options are increasing the cycle time and the cost of the electrophysiological procedure.

Embodiments of the present invention that are described hereinbelow provide improved techniques for performing both EA mapping and tissue ablation, using a single catheter having a controllable and/or adjustable size of the electrodes.

In some embodiments, a catheter configured for performing both EA mapping and tissue ablation comprises: (i) a shaft for insertion into an organ (e.g. a heart) of a patient, (ii) an expandable distal-end assembly, which is coupled to the shaft and comprises multiple splines, and (iii) a movable tube.

In some embodiments, at least a given spline among the multiple splines comprises an electrode that is configured to be placed in contact with tissue of the heart for performing the EA sensing and the tissue ablation.

In some embodiments, the tube is configured to be threaded over at least a portion of the expandable distal-end assembly, and is movable along a longitudinal axis of the distal-end assembly, for controlling at least the size, and optionally the shape, of a section of the electrode in contact with the tissue.

In some embodiments, the tube has a stopper for limiting the movement of the tube relative to the distal-end assembly, for example, between a proximal apex and a distal apex of the distal-end assembly.

In some embodiments, one or more additional electrodes are coupled to the tube and are electrically wired to the catheter, but are not intended to be electrically connected with any of the splines, or to be placed in contact with the tissue in question. In such embodiments, the catheter is configured to sense and/or to apply: (i) a unipolar signal between a selected electrode of the catheter (e.g., a section of a spline) in contact with the tissue and the additional electrode, and/or (ii) a bipolar signal between two selected electrodes (e.g., sections of two different spline) of the distal-end assembly.

In some embodiments, by moving the tube along the longitudinal axis of the distal-end assembly, a physician, or any other user of the catheter, may adjust the size of the electrode(s) used for the EA sensing and/or tissue ablation. For example, the physician may use: (i) the entire length of the spline (referred to herein as a large ablation electrode) for ablating a given area of the tissue, and (ii) a portion of the spline (i.e., an electrode smaller than the large ablation electrode) for sensing the EA signals in an area smaller than the given area.

In such embodiments, the physician may obtain high-resolution EA mapping (by using smaller electrodes). Subsequently, based on the EA mapping, the physician may increase the size of the electrode, e.g., by moving the tube distally along the longitudinal axis, for ablating the given area described above, or for ablating any other area of the tissue having a size typically larger than the area of the tissue that is covered, e.g., by the electrode, for the EA mapping.

The disclosed techniques improve the quality of electrophysiological procedures by adjusting the size of the electrode to correspond with the size required for the EA mapping and/or tissue ablation. Moreover, the disclosed techniques reduce the cycle time of electrophysiological procedures, by allowing a quick and simply adjustment of the electrode-size, and thereby, a quick switch between EA mapping of tissue and ablation of the tissue.

Furthermore, the disclosed techniques reduce the cost of electrophysiological procedures, by enabling the physician to carry out both sensing EA signals in tissue and ablating the tissue using a simple catheter that typically costs less than two different catheters, or a complex catheter having to or more different types of electrodes.

### SYSTEM DESCRIPTION

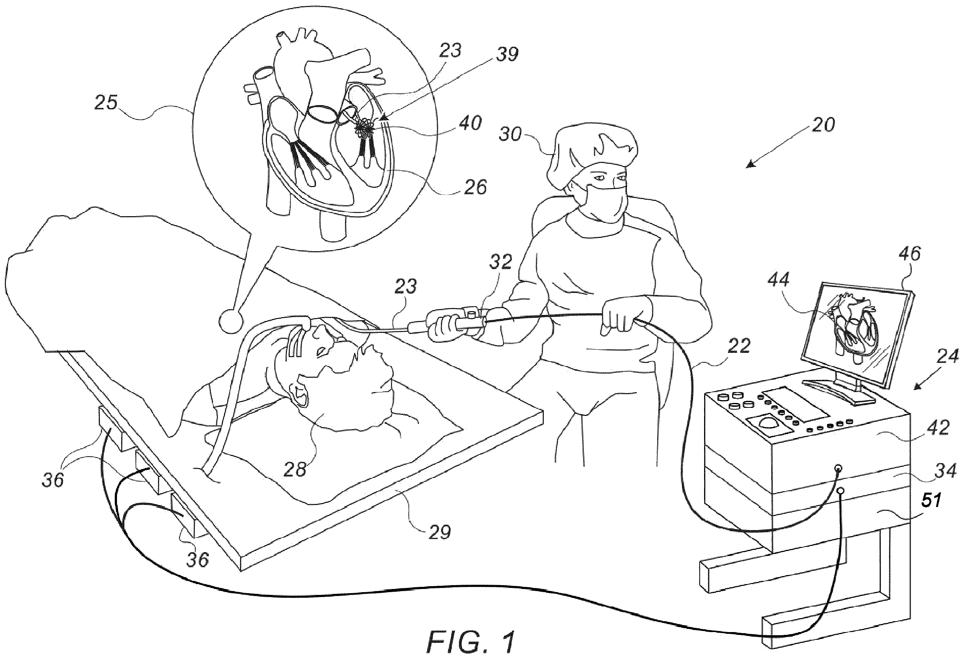

Fig. 1 is a schematic, pictorial illustration of a catheter-based position-tracking and ablation system 20, in accordance with an exemplary embodiment of the present invention. In some embodiments, system 20 comprises a catheter 22, in the present example an expandable cardiac catheter having a basket shape, and a control console 24. In the embodiment described herein, catheter 22 may be used for any suitable therapeutic and/or diagnostic purposes, such as but not limited to ablation of tissue in a heart 26.

In some embodiments, console 24 comprises a processor 42, typically a general-purpose computer, with suitable front end and interface circuits for receiving signals from catheter 22 and for controlling other components of system 20 described herein. Processor 42 may be programmed in software to carry out the functions that are used by the system, and is configured to store data for the software in a memory 51. The software may be downloaded to console 24 in electronic form, over a network, for example, or it may be provided on non-transitory tangible media, such as optical, magnetic or electronic memory media. Alternatively, some or all of the functions of processor 42 may be carried out using an application-specific integrated circuit (ASIC) or any suitable type of programmable digital hardware components.

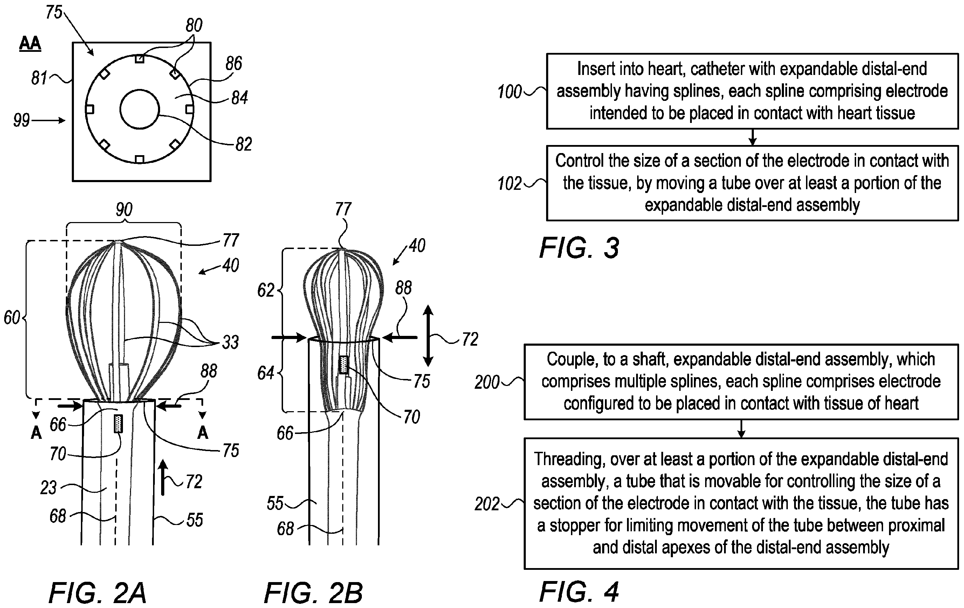

Reference is now made to an inset 25. In some embodiments, catheter 22 comprises a distal-end assembly 40 having multiple splines (shown in detail in Fig. 2 below), and a shaft 23 for inserting distal-end assembly 40 to a target location for ablating tissue in heart 26. During an ablation procedure, physician 30 inserts catheter 22 through the vasculature system of a patient 28 lying on a table 29. Physician 30 moves distal-end assembly 40 to the target location in heart 26 using a manipulator 32 near a proximal end of catheter 22, which is connected to interface circuitry of processor 42.

In some embodiments, catheter 22 comprises a position sensor 39 of a position tracking system, which is coupled to the distal end of catheter 22, e.g., in close proximity to distal-end assembly 40. In the present example, position sensor 39 comprises a magnetic position sensor, but in other embodiments, any other suitable type of position sensor (e.g., other than magnetic based) may be used.

Reference is now made back to the general view of Fig. 1. In some embodiments, during the navigation of distal-end assembly 40 in heart 26, processor 42 receives signals from magnetic position sensor 39 in response to magnetic fields from external field generators 36, for example, for the purpose of measuring the position of distal-end assembly 40 in heart 26. In some embodiments, console 24 comprises a driver circuit 34, configured to drive magnetic field generators 36. Magnetic field generators 36 are placed at known positions external to patient 28, e.g., below table 29.

In some embodiments, processor 42 is configured to display, e.g., on a display 46 of console 24, the tracked position of distal-end assembly 40 overlaid on an image 44 of heart 26.

The method of position sensing using external magnetic fields is implemented in various medical applications, for example, in the CARTO^{™} system, produced by Biosense Webster Inc. (Irvine, Calif.) and is described in detail in U.S. Patent Nos. 5,391,199, 6,690,963, 6,484,118, 6,239,724, 6,618,612 and 6,332,089, in PCT Patent Publication WO 96/05768, and in U.S. Patent Application Publication Nos. 2002/0065455 A1, 2003/0120150 A1 and 2004/0068178 A1.

### DISTAL-END ASSEMBLY HAVING ELECTRODES WITH ADJUSTABLE SIZE

Fig. 2A is a schematic, side view of distal-end assembly 40 and a movable tube 55, configured for controlling the size of one or more electrodes of distal-end assembly 40 that are placed in contact with tissue of heart 26, in accordance with an exemplary embodiment of the present invention.

In some embodiments, distal-end assembly 40 is coupled to shaft 23 and is navigated by physician 30 to be placed in contact with tissue in question of heart 26 or with tissue of any other organ of the patient, as described in Fig. 1 above.

In some embodiments, distal-end assembly 40 is expandable, in the present example having a basket shape, but in other embodiments, distal-end assembly 40 may have any other suitable type of an expandable shape.

In some embodiments, distal-end assembly 40 has splines 33 coupled between a proximal apex 66 and a distal apex 77 thereof. In the present example, at least one of and typically all splines 33 are made from nitinol or any other suitable biocompatible material. Splines 33 are electrically conductive and can be electrically shorted or isolated from one another, e.g., by apexes 66 and 77 or using any other suitable electrically insulating apparatus.

In other embodiments, one or more of splines 33 may have electrically isolated surfaces at both edges and an electrically conductive surface between the edges that may serve as an electrode. In such embodiments, at least one of apexes 66 and 77 may be electrically conductive.

In some embodiments, apexes 66 and 77 are movable relative to one another so as to expand and collapse distal-end assembly 40. For example, apex 66 is moved distally in a direction indicated by arrow 72, which is parallel to an axis 68 of catheter 22, so as to expand distal-end assembly 40. Similarly, apex 66 is moved proximally along axis 68, so as to collapse distal-end assembly 40. In the present example, axis 68 constitutes a longitudinal axis of both catheter 22 and distal-end assembly 40.

In some embodiments, distal-end assembly 40 may have two position sensors 39 coupled to, in adjacent to, apexes 66 and 77, respectively. Based on the position signals received from position sensors 39, processor 42 calculates the distance between apexes 66 and 77, and thereby, the expansion-level of distal-end assembly 40.

In some embodiments, tube 55 has a diameter 88, which may be fixed or altered along axis 68, and at least one electrode 70 coupled to the surface of the inner or outer perimeter of tube 55. Electrode 70 is configured to be used, for example, as a reference electrode for applying, to the tissue in question, a unipolar ablation signal between the electrode of a given spline 33 and electrode 70. Similarly, electrode 70 may be used as a reference electrode for sensing, in the tissue in question, a unipolar EA signal between the electrode of given spline 33 and electrode 70. Note that in such embodiments, electrode 70 is not intended to be placed in contact with the tissue in question and the electrode of given spline 33 is placed in contact with the tissue in question.

In some embodiments, tube 55 is configured to be threaded over at least a portion of shaft 23, and over at least a portion of distal-end assembly 40. Tube 55 is configured to be moved proximally and distally along axis 68, for controlling at least the size of a section described herein of the electrode, which is made from one or more splines 33, and is placed in contact with the tissue.

In some embodiments, tube 55 has a stopper 99 formed on an upper edge 75 of tube 55. Stopper 99, whose structure is described herein, is configured for limiting the movement of tube 55 relative to distal-end assembly 40.

In the example, of Fig. 2A, edge 75 of tube 55 is placed in contact with apex 66, so that stopper 99 is configured to limit tube 55 to move only distally in direction 72 parallel to axis 68.

Reference is now made to an inset 81, which is a top-view AA of upper edge 75 of tube 55.

In some embodiments, upper edge 75 of tube 55 has an inner perimeter 82 and an outer perimeter 86 defining a donut-shape. Note that a surface 84 between perimeters 82 and 86 comprises a solid surface, which is configured to limit the movement of upper edge 75 of tube 55 between apexes 66 and 77 as will be described in detail below. In the present example, stopper 99 is consisting of surface 84 and perimeters 82 and 86.

In some embodiments, the minimal diameter of stopper 99, e.g., the circle surrounded by inner perimeter 82, is larger than the diameter of shaft 23, so that tube 55 can be moved, over shaft 23 and at least a portion of distal-end assembly 40, along axis 68.

In some embodiments, upper edge 75 of tube 55 has channels 80 configured for leading splines 33 and for controlling the position of at least one of splines 33 along the inner surface of outer perimeter 86 of tube 55. In other words, channels 80 prevent slipping or shifting of one or more splines 33 along perimeter 86, which may result in undesired physical and electrical contact between two or more splines 33.

In some embodiments, when tube 55 is moved distally in direction 72, stopper 99 is configured to limit the movement of tube 55 such that upper edge 75 cannot be positioned distally to apex 77. In the present configuration, surface 84 collides into the upper edge section of splines 33 that are coupled to distal apex 77, and therefore, limits the distal movement of upper edge 75 of tube 55, beyond distal apex 77.

In the present example, stopper 99 is formed at upper edge 75 of tube 55. In other embodiments, stopper 99 may be formed at any other location along axis 68 of tube 55 and may have any suitable length, e.g., along axis 68. Note that in such configurations, the distal-most position of upper edge 75 relative to distal apex 77 may be different compared to the position described above. In alternative embodiments, stopper 99 may be eliminated from the configuration of tube 55, and/or may be implemented using any other suitable configuration.

Reference is now made back to the general view of Fig. 2A. In some embodiments, in the position shown in Fig. 2A, (i) distal-end assembly 40 is expanded, and (ii) tube 75 is positioned at its proximal-most position relative to distal-end assembly 40, such that edge 75 is placed in contact with apex 66. In this position, distal-end assembly 40 has a maximal length of a section 60 (whose size is provided below), and a maximal diameter 90 (e.g., about 12 mm). Therefore, the entire outer surface of splines 33 is exposed to heart 26, and one or more of splines 33 may be placed in contact with the tissue of heart 26. In other words, a maximal size of a section of the electrodes (e.g., splines 33) of distal-end assembly 40 may be placed in contact with the tissue.

In some embodiments, physician 30 may use system 20 for applying one or more ablation signals (e.g., ablation pulses) to one or more respective splines 33 that are placed in contact with the tissue, so as to form a lesion at one or more intended positions in the tissue.

In other embodiments, physician 30 may use system 20 for sensing one or more EA signals in the tissue, but the position shown in Fig. 2A is typically used for ablation, whereas smaller electrodes are typically used for sensing the EA signals.

In some embodiments, by moving tube 55 along distal-end assembly 40, physician 30 or any other used of system 20, may adjust or control the size of the electrode (e.g., a section of spline 33) placed in contact with the tissue of heart 26. Moreover, stopper 99 is configured to limit the movement of upper edge 75 of tube 55, between proximal apex 66 and distal apex 77 of the distal-end assembly 40.

Fig. 2B is a schematic, side view of distal-end assembly 40 and movable tube 55 for controlling the size of the electrodes of distal-end assembly 40 that are placed in contact with tissue of heart 26, in accordance with an embodiment of the present invention.

In some embodiments, physician 30 may use manipulator 32, or any other apparatus of system 20, for moving tube along axis 68, so as to adjust the size of the electrodes placed in contact with the tissue in question.

In some embodiments, at the position of tube 55 shown in Fig. 2B, tube 55 can be moved along axis 68, both proximally and distally, for adjusting the size of the section of splines 33 placed in contact with the tissue of heart 26. In the example of Fig. 2B, sections 62 and 64 have approximately the same length of section 60 shown in Fig. 2A above. For example, the length of section 60 may be between about 15 mm and 10 mm, the length of section 62 may be between about 6 mm and 3 mm, and the length of section 64, which is typically obtained by subtracting the length of section 62 from that of section 60, may be between about 12 mm and 7 mm. Note that in this configuration, tube 55 is positioned between section 64 and heart 26, so that only section 62 of the electrodes (e.g., splines 33) may be placed in contact with the tissue in question of heart 26.

In the context of the present disclosure and in the claims, the terms "about" or "approximately" for any numerical values or ranges indicate a suitable dimensional tolerance that allows the part or collection of components to function for its intended purpose as described herein. Moreover, the terms "about" or "approximately" may also be used for comparing between a physical dimension, or a measurable feature of two or more elements, and the terms "about" or "approximately" may indicate a suitable dimensional tolerance between the compared elements.

In some embodiments, by reducing the size of the section of splines 33 placed in contact with the tissue (e.g., section 62), physician 30 may use system 20 for sensing one or more EA signals in the tissue. Note that typically, but not necessarily, smaller electrodes (e.g., section 62 of splines 33) are preferred for sensing the EA signals in the tissue in question, and larger electrodes (e.g., section 60 of splines 33 shown in Fig. 2A above) are preferred for ablating the tissue in question.

In other embodiments, physician 30 may use manipulator 32 for adjusting the distance between apexes 66 and 77. For example, when tube 55 is in the position shown in Fig. 2B, physician 30 may move apex 66 proximally along axis 68, so as to partially-collapse distal-end assembly 40, and thereby reduce the size of diameter 90 and increase the length of distal-end assembly 40.

In some embodiments, the movement of tube 55 and apex 66 (relative to apex 77) may be carried out independently, so as to provide physician 30 with an operational flexibility of catheter 22.

The disclosed techniques enable the incorporation of one set of electrodes on the same splines 33 of a basket catheter (e.g., distal-end assembly 40). The electrodes can serve both for ablating tissue and for sensing EA signals, by controlling and adjusting the size of the section of splines 33 that are placed in contact with the tissue of heart 26. Fig. 3 is a flow chart that schematically illustrates a method, which is not claimed (but is considered as useful for understanding the invention), for controlling the size of one or more electrodes of expandable distal-end assembly 40 that are placed in contact with tissue of heart 26.

The method begins at a catheter insertion step 100 with physician 30 inserting into heart 30 distal-end assembly 40 of catheter 22, as described in Fig. 1 above. In some embodiments, distal-end assembly 40 has multiple splines 33, at least one of, and typically all splines 33, are made from an electrically conductive alloy, so that the entire surface, or a large portion, of each spline 33 serves as an electrode intended to be placed in contact with tissue of heart 26, as also described in detail in Figs. 2A and 2B above.

At an electrode size adjustment step 102 that terminates the method, physician 30 controls the size of a section of the one or more electrodes in contact with tissue, by moving tube 55, along axis 68, over at least a portion of distal-end assembly 40. In the example of Fig. 2A, physician 30 exposes section 60, which is the entire length of one or more splines 33 (that serve as the electrodes) in contact with the tissue, e.g., for ablating the tissue of heart 26. In the example of Fig. 2B, physician 30 moves tube 50 toward the distal-end of distal-end assembly 40. In this configuration, tube 55 covers section 64, and only section 62 of splines 33 may be placed in contact with the tissue of heart 26. Note that section 60 of Fig. 2A has a length approximately similar to a combined length of sections 62 and 64 of Fig. 2B. Thus, section 62, which is smaller than section 60, may be used for sensing EA signals in the tissue of heart 26, or for any other suitable operation.

In some embodiments, the movement stroke of tube 55 relative to distal-end assembly 40, is limited by using stopper 99, which is described in detail in Fig. 2A above, or by using any other type of suitable apparatus. In the example of stopper 99, the stroke of upper edge 75 of tube 55 is limited between proximal apex 66 and distal apex 77 as described in detail in Figs. 2A and 2B.

In some embodiments, the EA sensing and/or signal ablating may be: (i) bipolar, e.g., between two splines 33 in contact with the tissue, or (ii) unipolar, e.g., between electrode 70 coupled to tube 55, as described in Fig. 2A above.

Fig. 4 is a flow chart that schematically illustrates a method for producing catheter 22 comprising expandable distal-end assembly 40 having splines 33 that serve as electrodes, and tube 55 for controlling the size of the electrodes placed in contact with tissue, in accordance with an embodiment of the present invention.

The method begins at a distal-end assembly coupling step 200, with coupling to shaft 23, expandable distal-end assembly 40 (e.g., having a basket shape), which comprises multiple splines 33. At least one of, and typically each spline 33, comprises at least an electrode configured to be placed in contact with tissue of heart 26, e.g., for sensing EA signals and/or for applying ablation signals to the tissue.

At a tube assembling step 202, tube 55 is threaded over at least a portion of expandable distal-end assembly 40. As described in Figs. 2A and 2B above, tube 55 is movable along axis 68 of distal-end assembly 40, for controlling the size of a section of the electrode in contact with the tissue. For example, section 60 of Fig. 2A above, or section 62 of Fig. 2B above.

In some embodiments, tube 55 has stopper 99 for limiting the movement of tube 55 between proximal axes 66 and distal apex 77 of distal-end assembly 40. Tube 55 may also have channels 80 for controlling (e.g., fixing) the position of splines 33 along the surface of outer perimeter 86 of upper edge 75, as described in Fig. 2A above.

In the present example, distal-end assembly 40 as eight splines 33 and tube 55 has eight channels 80, one for spline 33. In other configurations, distal-end assembly 40 may have any other suitable number of splines 33 and tube 55 may have any suitable number of channels 80, which may be similar to or different from the number of splines 33 of distal-end assembly 40.

In some embodiments, the method may comprise coupling of one or more electrodes 70 to tube 55, so as to enable the application of unipolar ablation signals and the sensing of unipolar EA signals described in detail in Fig, 2A above.

In some embodiments, electrode 70 may be coupled to the inner or outer surface of tube 55, as described in Fig. 2A above, or may be embedded within the wall of tube 55. Moreover, an electrical lead or trace may be produced for electrically connecting between electrode 70 and catheter 22. Note that electrode 70 and the lead or trace are not intended to be placed in contact with the tissue in question, so as to enable the unipolar signal application and/or sensing.

Although the embodiments described herein mainly address electrophysiological procedures in patient heart (e.g., EA sensing and tissue ablation), the methods and systems described herein can also be used in other applications, such as in electrophysiological procedures carried out on other organs of a patient, and in electrophysiological procedures carried out in patient lung(s) and in treatment of renal denervation.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. The invention is defined by the following claims.

## Claims

1. A catheter with an adjustable size electrode, the catheter comprising:
a shaft for insertion into an organ of a patient;
an expandable distal-end assembly, which is coupled to the shaft and comprises multiple splines, wherein at least a given spline among the multiple splines comprises an electrode that is configured to be placed in contact with tissue of the organ; and
a tube, which is configured to be threaded over at least a portion of the expandable distal-end assembly, and is movable along the distal-end assembly for controlling at least a size of a section of the electrode in contact with the tissue, wherein the tube has a stopper for limiting a movement of the tube relative to the distal-end assembly.

2. The catheter according to claim 1, wherein the tube has at least a channel for controlling a position of at least the given spline along a perimeter of the tube.

3. The catheter according to claim 1, wherein the splines are coupled between a distal apex and a proximal apex of the distal-end assembly, and wherein the tube is configured to be moved: (i) toward the proximal apex for increasing the size of the section, and (ii) toward the distal apex for reducing the size of the section.

4. The catheter according to claim 3, wherein the stopper is configured to limit the movement of the tube between the proximal apex and the distal apex of the distal-end assembly.

5. The catheter according to claim 3, wherein at least the given spline is electrically conductive and the electrode is configured to perform one or both of: (i) applying an ablation signal to the tissue, and (ii) sensing an electro-anatomic (EA) signal in the tissue.

6. The catheter according to claim 5, wherein at least the electrode is configured to: (i) apply the ablation signal when the size of the section is increased, and (ii) sense the EA signal when the size of the section is reduced.

7. The catheter according to claim 5, and comprising an additional electrode, which is coupled to the tube and is not intended to be placed in contact with the tissue, and wherein the distal-end assembly is configured to apply a unipolar ablation signal between the electrode and the additional electrode.

8. The catheter according to claim 5, and comprising an additional electrode, which is coupled to the tube and is not intended to be placed in contact with the tissue, and wherein the distal-end assembly is configured to sense a unipolar EA signal between the electrode and the additional electrode.

9. A method for producing a catheter, the method comprising:
coupling, to a shaft, an expandable distal-end assembly, which comprises multiple splines, wherein at least a given spline among the multiple splines comprises an electrode that is configured to be placed in contact with tissue of an organ of a patient; and
threading, over at least a portion of the expandable distal-end assembly, a tube that is movable for controlling at least a size of a section of the electrode in contact with the tissue, wherein the tube has a stopper for limiting a movement of the tube relative to the distal-end assembly.

10. The method according to claim 9, wherein the tube has at least a channel for controlling a position of at least one of the splines along a perimeter of the tube.

11. The method according to claim 9, wherein the splines are coupled between a distal apex and a proximal apex of the distal-end assembly, and wherein the stopper is configured to limit the movement of the tube between the distal apex and the proximal apex for controlling at least the size of the section.

12. The method according to claim 9, and comprising coupling to the tube an additional electrode, which is not intended to be placed in contact with the tissue, so as to one or both: (i) apply a unipolar ablation signal between the electrode and the additional electrode, and (ii) sense a unipolar EA signal between the electrode and the additional electrode.

## Patentansprüche

1. Katheter mit einer Elektrode mit einstellbarer Größe, der Katheter umfassend:
einen Schaft für ein Einführen in ein Organ eines Patienten;
eine erweiterbare distalendige Baugruppe, die mit dem Schaft gekoppelt ist und mehrere Keilwellenprofile umfasst, wobei mindestens ein gegebenes Keilwellenprofil unter den mehreren Keilwellenprofilen eine Elektrode umfasst, die konfiguriert ist, um mit Gewebe des Organs in Kontakt gebracht zu werden; und
ein Rohr, das konfiguriert ist, um über mindestens einen Anteil der erweiterbaren distalendigen Baugruppe geschraubt zu werden und entlang der distalendigen Baugruppe zum Steuern mindestens einer Größe eines Abschnitts der Elektrode bewegbar ist, die mit dem Gewebe in Kontakt steht, wobei das Rohr einen Stopper zum Begrenzen einer Bewegung des Rohrs relativ zu der distalendigen Baugruppe aufweist.

2. Katheter nach Anspruch 1, wobei das Rohr mindestens einen Kanal zum Steuern einer Position von mindestens dem gegebenen Keilwellenprofil entlang eines Umfangs des Rohrs aufweist.

3. Katheter nach Anspruch 1, wobei die Keilwellenprofile zwischen einem distalen Scheitelpunkt und einem proximalen Scheitelpunkt der distalendigen Baugruppe gekoppelt sind und wobei das Rohr konfiguriert ist, um bewegt zu werden: (i) in Richtung des proximalen Scheitelpunkts zum Erhöhen der Größe des Abschnitts, und (ii) in Richtung des distalen Scheitelpunkts zum Verringern der Größe des Abschnitts.

4. Katheter nach Anspruch 3, wobei der Stopper konfiguriert ist, um die Bewegung des Rohrs zwischen dem proximalen Scheitelpunkt und dem distalen Scheitelpunkt der distalendigen Baugruppe zu begrenzen.

5. Katheter nach Anspruch 3, wobei mindestens das gegebene Keilwellenprofil elektrisch leitfähig ist und die Elektrode konfiguriert ist, um eines oder beides auszuführen von: (i) Anwenden eines Ablationssignals auf das Gewebe und (ii) Erfassen eines elektroanatomischen (EA) Signals in dem Gewebe.

6. Katheter nach Anspruch 5, wobei mindestens die Elektrode konfiguriert ist zum: (i) Anwenden des Ablationssignals, wenn die Größe des Abschnitts erhöht wird, und (ii) Erfassen des EA-Signals, wenn die Größe des Abschnitts verringert wird.

7. Katheter nach Anspruch 5, und umfassend eine zusätzliche Elektrode, die mit dem Rohr gekoppelt ist und nicht dafür gedacht ist, mit dem Gewebe in Kontakt gebracht zu werden, und wobei die distalendige Baugruppe konfiguriert ist, um ein unipolares Ablationssignal zwischen der Elektrode und der zusätzlichen Elektrode anzuwenden.

8. Katheter nach Anspruch 5, und umfassend eine zusätzliche Elektrode, die mit dem Rohr gekoppelt ist und nicht dafür gedacht ist, mit dem Gewebe in Kontakt gebracht zu werden, und wobei die distalendige Baugruppe konfiguriert ist, um ein unipolares EA-Signal zwischen der Elektrode und der zusätzlichen Elektrode zu erfassen.

9. Verfahren zum Produzieren eines Katheters, das Verfahren umfassend:
Koppeln einer erweiterbaren distalendigen Baugruppe, die mehrere Keilwellenprofile umfasst, mit einem Schaft, wobei mindestens ein gegebenes Keilwellenprofil unter den mehreren Keilwellenprofilen eine Elektrode umfasst, die konfiguriert ist, um mit dem Gewebe eines Organs eines Patienten in Kontakt gebracht zu werden; und
Schrauben, über mindestens einen Anteil der erweiterbaren distalendigen Baugruppe, eines Rohrs, das zum Steuern mindestens einer Größe eines Abschnitts der Elektrode bewegbar ist, die mit dem Gewebe in Kontakt steht, wobei das Rohr einen Stopper zum Begrenzen einer Bewegung des Rohrs relativ zu der distalendigen Baugruppe aufweist.

10. Verfahren nach Anspruch 9, wobei das Rohr mindestens einen Kanal zum Steuern einer Position von mindestens einem der Keilwellenprofile entlang eines Umfangs des Rohrs aufweist.

11. Verfahren nach Anspruch 9, wobei die Keilwellenprofile zwischen einem distalen Scheitelpunkt und einem proximalen Scheitelpunkt der distalendigen Baugruppe gekoppelt sind, und wobei der Stopper konfiguriert ist, um die Bewegung des Rohrs zwischen dem distalen Scheitelpunkt und dem proximalen Scheitelpunkt zum Steuern mindestens der Größe des Abschnitts zu begrenzen.

12. Verfahren nach Anspruch 9, und umfassend das Koppeln einer zusätzlichen Elektrode, die nicht dafür gedacht ist, mit dem Gewebe in Kontakt gebracht zu werden, an das Rohr, um eines oder beides von: (i) Anwenden eines unipolaren Ablationssignals zwischen der Elektrode und der zusätzlichen Elektrode und (ii) Erfassen eines unipolaren EA-Signals zwischen der Elektrode und der zusätzlichen Elektrode.

## Revendications

1. Cathéter avec une électrode de taille ajustable, le cathéter comprenant :
une tige destinée à être insérée dans un organe d'un patient ;
un ensemble extrémité distale extensible, lequel est accouplé à la tige et comprend plusieurs cannelures, dans lequel au moins une cannelure donnée parmi les cannelures comprend une électrode qui est conçue pour être placée en contact avec du tissu de l'organe ; et
un tube, lequel est conçu pour être enfilé sur au moins une partie de l'ensemble extrémité distale extensible, et peut être déplacé le long de l'ensemble extrémité distale pour contrôler au moins une taille d'une section de l'électrode en contact avec le tissu, dans lequel le tube a un bouchon destiné à limiter un déplacement du tube par rapport à l'ensemble extrémité distale.

2. Cathéter selon la revendication 1, dans lequel le tube a au moins un canal destiné à contrôler une position d'au moins la cannelure donnée le long d'un périmètre du tube.

3. Cathéter selon la revendication 1, dans lequel les cannelures sont accouplées entre un apex distal et un apex proximal de l'ensemble extrémité distale, et dans lequel le tube est conçu pour être déplacé : (i) vers l'apex proximal pour augmenter la taille de la section, et (ii) vers l'apex distal pour réduire la taille de la section.

4. Cathéter selon la revendication 3, dans lequel le bouchon est conçu pour limiter le déplacement du tube entre l'apex proximal et l'apex distal de l'ensemble extrémité distale.

5. Cathéter selon la revendication 3, dans lequel au moins la cannelure donnée est électriquement conductrice et l'électrode est configurée pour effectuer l'une des opérations suivantes, ou les deux : (i) appliquer un signal d'ablation au tissu, et (ii) détecter un signal électroanatomique (EA) dans le tissu.

6. Cathéter selon la revendication 5, dans lequel au moins l'électrode est configurée pour : (i) appliquer le signal d'ablation lorsque la taille de la section est augmentée, et (ii) détecter le signal EA lorsque la taille de la section est réduite.

7. Cathéter selon la revendication 5, et comprenant une électrode supplémentaire, laquelle est accouplée au tube et n'est pas prévue pour être placée en contact avec le tissu, et dans lequel l'ensemble extrémité distale est configuré pour appliquer un signal d'ablation unipolaire entre l'électrode et l'électrode supplémentaire.

8. Cathéter selon la revendication 5, et comprenant une électrode supplémentaire, laquelle est accouplée au tube et n'est pas prévue pour être placée en contact avec le tissu, et dans lequel l'ensemble extrémité distale est configuré pour détecter un signal EA unipolaire entre l'électrode et l'électrode supplémentaire.

9. Procédé destiné à produire un cathéter, le procédé comprenant :
l'accouplement, à une tige, d'un ensemble extrémité distale extensible, lequel comprend plusieurs cannelures, dans lequel au moins une cannelure donnée parmi les cannelures comprend une électrode qui est conçue pour être placée en contact avec du tissu d'un organe d'un patient ; et
l'enfilage, sur au moins une partie de l'ensemble extrémité distale extensible, d'un tube qui peut être déplacé pour contrôler au moins une taille d'une section de l'électrode en contact avec le tissu, dans lequel le tube a un bouchon destiné à limiter un déplacement du tube par rapport à l'ensemble extrémité distale.

10. Procédé selon la revendication 9, dans lequel le tube a au moins un canal destiné à contrôler une position d'au moins l'une des cannelures le long d'un périmètre du tube.

11. Procédé selon la revendication 9, dans lequel les cannelures sont accouplées entre un apex distal et un apex proximal de l'ensemble extrémité distale, et dans lequel le bouchon est conçu pour limiter le déplacement du tube entre l'apex distal et l'apex proximal pour contrôler au moins la taille de la section.

12. Procédé selon la revendication 9, et comprenant l'accouplement au tube d'une électrode supplémentaire, laquelle n'est pas prévue pour être placée en contact avec le tissu, de façon à l'une des opérations suivantes, ou les deux : (i) appliquer un signal d'ablation unipolaire entre l'électrode et l'électrode supplémentaire, et (ii) détecter un signal EA unipolaire entre l'électrode et l'électrode supplémentaire.
